# EUROPEAN PATENT APPLICATION

(11) **EP 4 461 232 A1**
(43) Date of publication of application: **13.11.2024**
(21) Application number: 22902482.3
(22) Date of filing: 10.03.2022
(51) Int. Cl.: A61B 8/14, A61N 7/02, A61B 17/00

(54) **DISPLAY DEVICE FOR HIFU THERAPY**

(30) Priority: 07.12.2021 JP 2021198691
(71) Applicant: SONIRE THERAPEUTICS INC., Tokyo 160-0023 (JP)
(72) Inventor: URAYAMA Hiroaki, Tokyo 160-0023 (JP); OKAMOTO Jun, Tokyo 160-0023 (JP)
(74) Representative: Novagraaf International SA
(86) International application number: PCT/JP2022/010562
(87) International publication number: WO 2023/105809

(57) **Abstract**

The present invention addresses the problem of simplifying an HIFU therapy in which an operation is performed a plurality of times. The present invention is a display device for an HIFU therapy, the display device being intended to perform image acquisition processing for acquiring a first ultrasound image that shows the condition of a patient before a first-round operation and a second ultrasound image that shows the condition of the patient before a second-round operation, image production processing for producing a superimposed image in which the first ultrasound image and the second ultrasound image are superimposed on each other, and display processing for displaying the superimposed image.

## Description

### TECHNICAL FIELD

The present invention relates to a HIFU therapeutic display apparatus and, in particular, relates to a technique for displaying an ultrasound image that represents how a patient is treated.

### BACKGROUND

Therapeutic devices employing high intensity focused ultrasound therapy are widely used. Such therapeutic devices are referred to as HIFU (High Intensity Focused Ultrasound) irradiation devices or HIFU irradiation systems, which irradiate a treatment site with ultrasound waves, thereby necrotizing a tissue.

Typically, a HIFU irradiation device includes a plurality of ultrasound transducers that are disposed on a bowl-shaped surface. The plurality of ultrasound transducers are disposed such that a point is irradiated with ultrasound waves emanating from the respective transducers, forming a focus. For a medical treatment, the focal point is adjusted to be located at a treatment site, which is irradiated with ultrasound waves. The location of irradiation is confirmed using an ultrasound diagnostic apparatus that shows a focal point on an ultrasound image.

Patent Document 1 listed below discloses an ultrasound therapeutic apparatus in which the location of a focal point is observed using an ultrasound diagnostic apparatus that displays a B-mode image (tomographic image). This apparatus emits weak ultrasound waves at a level with no effect on tissues from therapeutic ultrasound transducers and displays a tomographic image through transmission and reception of ultrasound waves using an ultrasound imaging probe. As a specimen tissue has acoustic characteristics that vary with change in temperature of the tissue, the tomographic image indicates the location of the focal point by the brightness level.

Patent Document 2 discloses an ultrasound therapeutic apparatus which detects a location irradiated with therapeutic ultrasound waves using an ultrasound probe, and which indicates the location irradiated with ultrasound waves on a three-dimensional image obtained by MRI (Magnetic Resonance Imaging) or CT (Computed Tomography).

### CITATION LIST

### PATENT LITERATURE

Patent Document 1: JP H08-71069 A
Patent Document 2: JP H11-313833 A

### SUMMARY

### TECHNICAL PROBLEM

High intensity focused ultrasound therapy (hereinafter referred to as HIFU therapy) is sometimes performed through treatment sessions in which a lesion is irradiated with ultrasound waves twice with a time interval of a few days to one week or so. In this case, states of the lesion are compared between before and after the first treatment session using B-mode images displayed by an ultrasound diagnostic apparatus, and then, for example, the irradiation location for the second session is set. However, the process of such comparison may involve complicated operations to display images.

The present invention is directed toward making HIFU therapy with multiple treatment sessions easier.

### SOLUTION TO PROBLEM

According to an aspect of the present invention, there is provided a HIFU therapeutic display apparatus for use in HIFU therapy, the HIFU therapeutic display apparatus being configured to execute image obtaining processing of obtaining a first ultrasound image representing a state of a patient before a first treatment session and a second ultrasound image representing a state of the patient before a second treatment session; image generation processing of generating a superimposed image having the first ultrasound image and the second ultrasound image laid over each other; and display processing of displaying the superimposed image.

Preferably, the display processing comprises processing of displaying the superimposed image and one of the first ultrasound image and the second ultrasound image adjacent to each other.

Preferably, the image generation processing comprises processing of adjusting transparency of one of the first ultrasound image and the second ultrasound image in response to user operation and then generating the superimposed image.

According to another aspect of the present invention, there is provided a HIFU therapeutic display apparatus for use in HIFU therapy, the HIFU therapeutic display apparatus being configured to execute image obtaining processing of obtaining a first ultrasound image representing a state of a patient before a first treatment session and a second ultrasound image representing a state of the patient before a second treatment session; and display processing of displaying at least one of an image with a graphical indication of an ultrasound irradiation location laid over the first ultrasound image and an image with a graphical indication of an ultrasound irradiation location laid over the second ultrasound image.

Preferably, the HIFU therapeutic display apparatus is configured to sequentially generate ultrasound images with lapse of time based on ultrasound waves transmitted and received by an ultrasound probe; and execute adjustment display processing of sequentially displaying the ultrasound images sequentially generated with lapse of time together with a graphical indication of a reference location of the ultrasound probe with lapse of time. The HIFU therapeutic display apparatus comprises a carrier mechanism configured to adjust the reference location in response to user operation while the adjustment display processing is being executed. Each of the first ultrasound image and the second ultrasound image is a two-dimensional image generated from a corresponding one of three-dimensional images. The three-dimensional images from which the first ultrasound image and the second ultrasound image are generated are generated based on ultrasound waves transmitted and received by the ultrasound probe that is transported for scanning at the reference location.

Preferably, the HIFU therapeutic display apparatus is configured to display a plurality of process indicators regarding operation for generating one of the first ultrasound image and the second ultrasound image; and a prompt for each of the plurality of process indicators.

Preferably, each of the first ultrasound image and the second ultrasound image is a two-dimensional image generated from a three-dimensional image generated based on ultrasound waves transmitted and received by an ultrasound probe that is transported for scanning, and the image obtaining processing comprises processing of generating, from the three-dimensional image from which the first ultrasound image is generated and the three-dimensional image from which the second ultrasound image is generated, the first ultrasound image and the second ultrasound image on a common plane in the patient.

Preferably, the HIFU therapeutic display apparatus is configured to display a plurality of process indicators regarding operation for the image generation processing and the display processing; and a prompt for each of the plurality of process indicators.

### ADVANTAGEOUS EFFECTS OF INVENTION

The present invention makes HIFU therapy with multiple treatment sessions easier.

### BRIEF DESCRIPTION OF DRAWINGS

[FIG. 1]

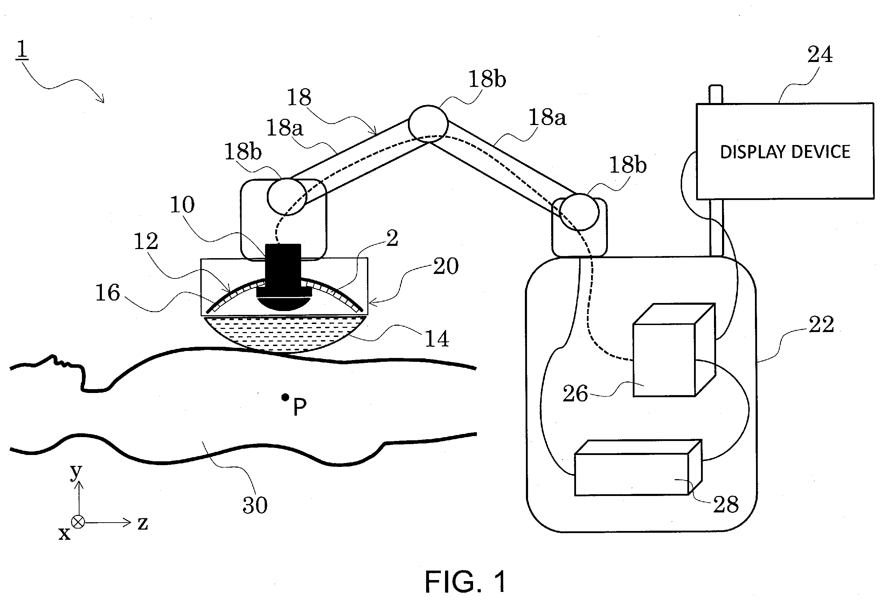

   FIG. 1 illustrates a configuration of a HIFU irradiation system according to an embodiment of the present invention.
[FIG. 2]

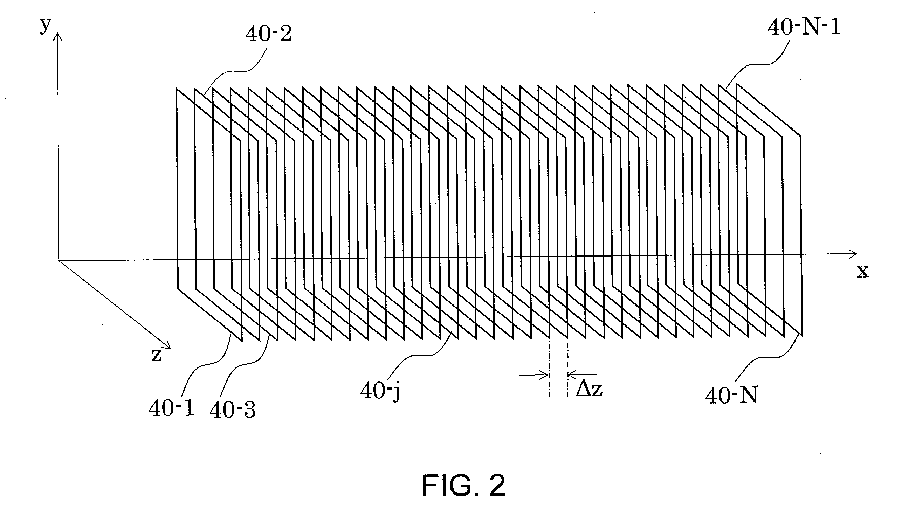

   FIG. 2 conceptually illustrates a three-dimensional image.
[FIG. 3]

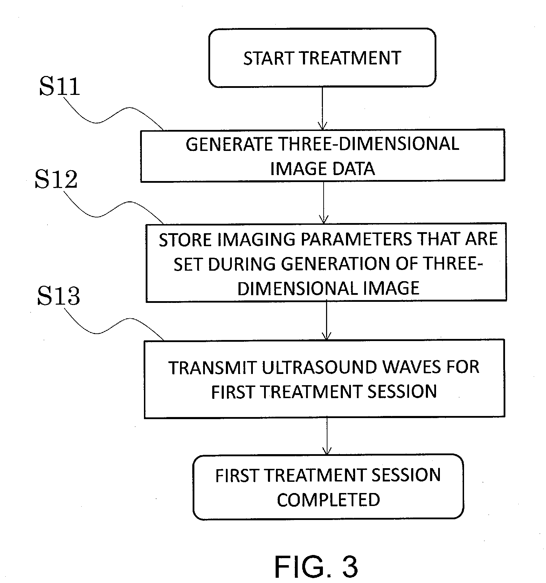

   FIG. 3 is a flowchart of a procedure for a first treatment session.
[FIG. 4]

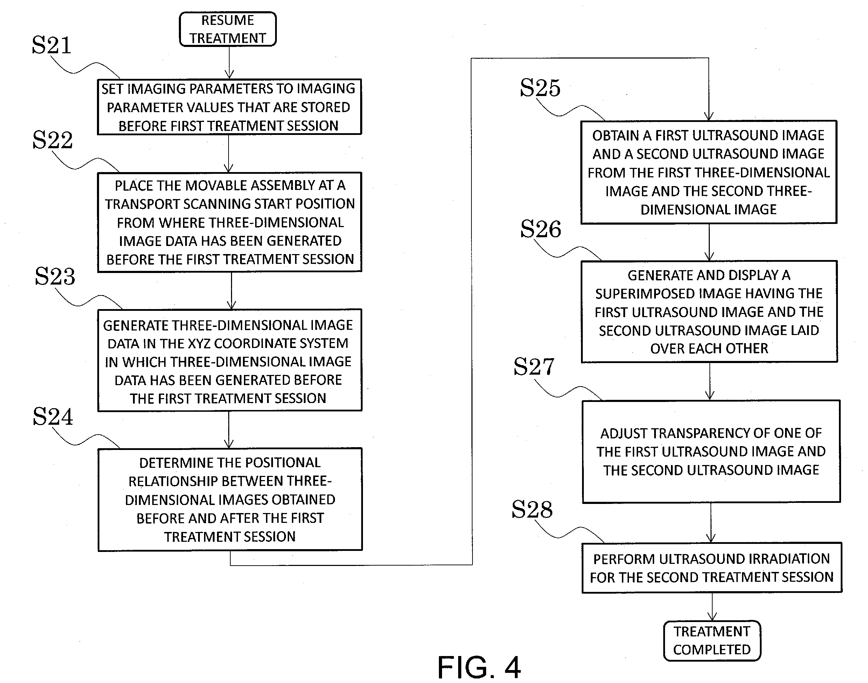

   FIG. 4 is a flowchart of a procedure for a second treatment session.
[FIG. 5]

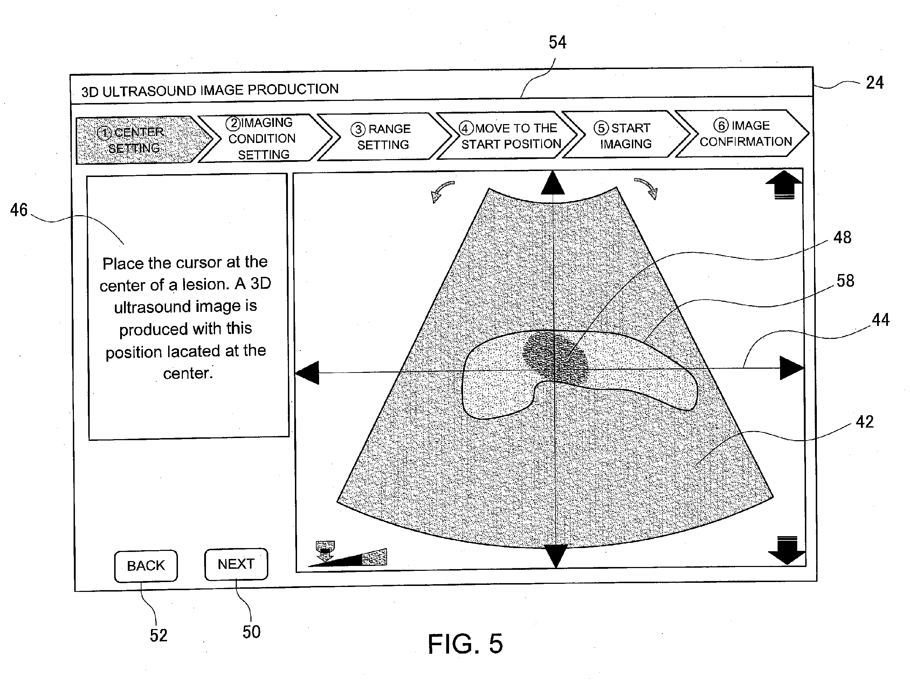

   FIG. 5 illustrates, by way of example, an image that is displayed on a display device during generation of three-dimensional image data.
[FIG. 6]

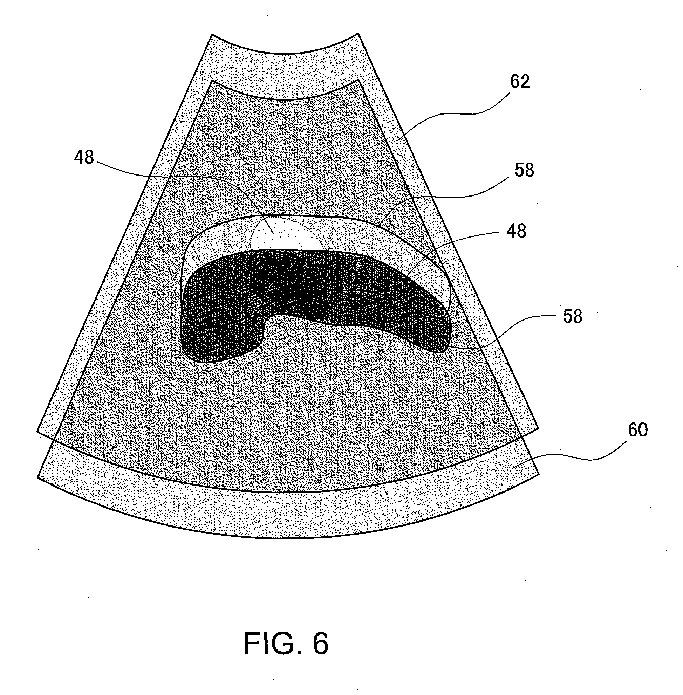

   FIG. 6 illustrates, by way of example, a superimposed image.
[FIG. 7]

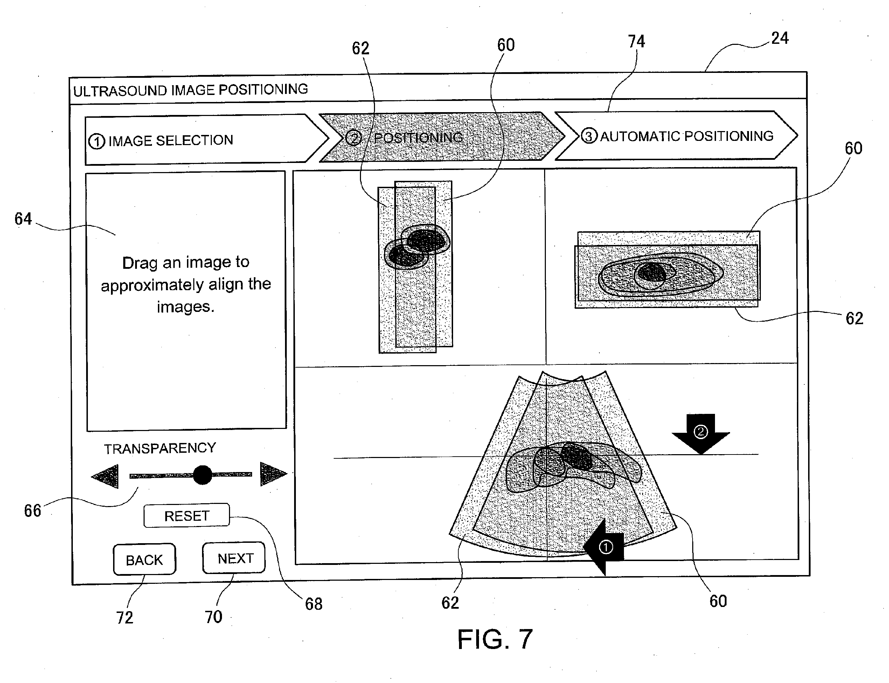

   FIG. 7 illustrates, by way of example, an image that is displayed on the display device while the location of a first ultrasound image is being adjusted.
[FIG. 8]

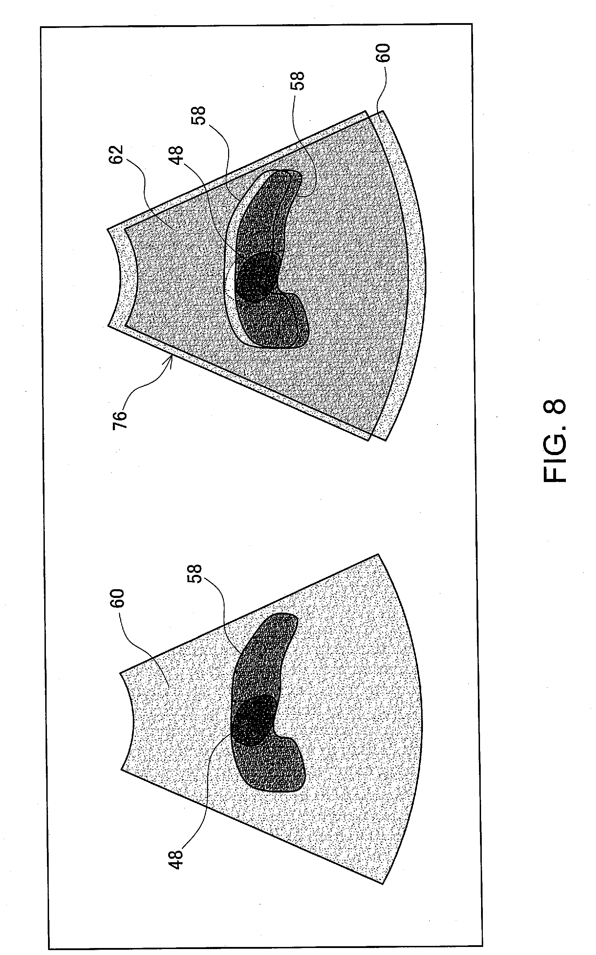

   FIG. 8 illustrates a manner in which the first ultrasound image and the superimposed image are displayed adjacent to each other for ease of comparison.
[FIG. 9]

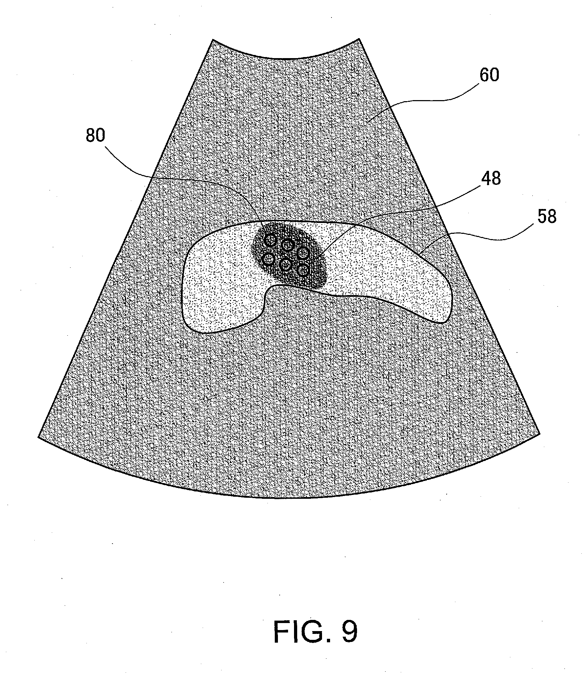

   FIG. 9 illustrates an image in which a graphical indication of an ultrasound irradiation location is laid over the first ultrasound image.
[FIG. 10]

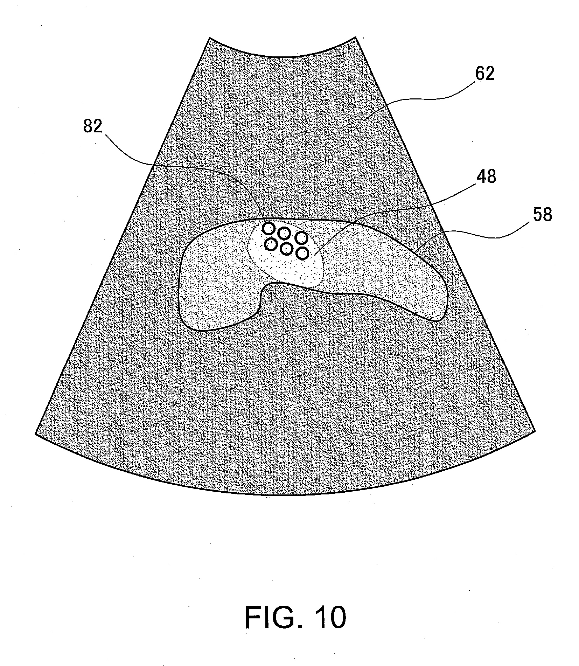

   FIG. 10 illustrates an image in which a graphical indication of an ultrasound irradiation location is laid over a second ultrasound image.

### DESCRIPTION OF EMBODIMENTS

Embodiments of the present invention will be described below with reference to the drawings. The same components illustrated in two or more drawings are denoted by the same reference numerals, and the description thereof is not repeated herein. The term "image data A" used herein, a combination of the term "image data" with a specific name "A", represents data for displaying an image A. Expressions of "output the image A" or otherwise process the image A in some manner represent processing of the image data A in some manner.

FIG. 1 illustrates a configuration of a HIFU irradiation system 1 according to an embodiment of the present invention. The HIFU irradiation system 1 includes an ultrasound probe 10, a HIFU transducer unit 12, a water bag 14, a robot arm 18, a controller 22, and a display device 24. In FIG. 1, the direction away from the head of a patient 30 toward the feet is oriented in a z-axis positive direction, and the upward direction is oriented in a y-axis positive direction. Further, the direction away from the right hand of the patient 30 toward the left hand is oriented in an x-axis positive direction.

The HIFU transducer unit 12 includes a bowl-shaped transducer housing 16 with an opening facing downward, and a plurality of ultrasound transducers 2 fixed to the transducer housing 16. The transducer housing 16 may have a shape that is similar to the shape of the side surface of a cone. The term cone here refers to a three-dimensional geometric shape consisting of a set of straight lines extending from a point in a space to a base. The ultrasound transducers 2 are fixed to the transducer housing 16 so as to provide an increased intensity of ultrasound waves at a treatment reference point P below the transducer housing 16 when the ultrasound transducers 2 emit ultrasound waves.

The ultrasound probe 10 is attached to the transducer housing 16 so as to enable transmission and reception of ultrasound waves at a position below the transducer housing 16 and above the treatment reference point P. In this embodiment, the ultrasound probe 10 penetrates a top portion of the transducer housing 16 in the vertical direction, and the tip portion that transmits and receives ultrasound waves points downward. The ultrasound probe 10 may be movable in the vertical direction. The ultrasound probe 10 may also be rotatable about the longitudinal axis. For therapy with multiple treatment sessions, the ultrasound probe 10 may be located at the same rotational and vertical position in every treatment session.

The water bag 14 is provided below the HIFU transducer unit 12 to match the acoustic impedance between the ultrasound transducers 2 and the patient 30 and between the ultrasound probe 10 and the patient 30. The water bag 14 may be a water-filled bag or a bag filled with some other substance.

The ultrasound probe 10, the HIFU transducer unit 12, and the water bag 14 are attached to the tip portion of the robot arm 18 serving as the carrier mechanism. The robot arm 18 is composed of a plurality of arms 18a connected by one or more joints 18b and is attached to a housing of the controller 22 via a joint 18b. As the arms 18a swing about the respective joints 18b serving as the axes of rotation, the robot arm 18 transports a movable assembly 20 including the ultrasound probe 10, the HIFU transducer unit 12, and the water bag 14 in three directions, x-axis, y-axis, and z-axis directions.

The controller 22 includes a HIFU control unit 26 and a robot control unit 28. The HIFU control unit 26 includes a computer, an electric circuit for controlling the ultrasound probe 10, and an electric circuit for controlling the HIFU transducer unit 12. The computer in the HIFU control unit 26 may be, for example, a business computer, a personal computer, or a tablet computer. The computer executes a program, thereby executing processing for generating images or processing for displaying images. One or more operation devices (not illustrated) that enable a user to operate the HIFU irradiation system 1 are connected to the HIFU control unit 26. Examples of such operation devices may include a mouse, a touch panel integrated with the display device 24, a switch, and a keyboard.

The HIFU control unit 26 executes processing for actuating the ultrasound probe 10 and the HIFU transducer unit 12. For example, the HIFU control unit 26 causes the ultrasound probe 10 to transmit and receive ultrasound waves, generates B-mode image data based on a reception signal that is based on the ultrasound waves received by the ultrasound probe 10, and causes the display device 24 to display a B-mode image. The HIFU control unit 26 further causes the ultrasound transducers 2 included in the HIFU transducer unit 12 to transmit therapeutic ultrasound waves.

The robot control unit 28 may control the robot arm 18 in accordance with control from the HIFU control unit 26 to cause the robot arm 18 to transport the movable assembly 20. The robot control unit 28 may include an operation device. In this case, the robot control unit 28 may control the robot arm 18 in response to user operation of the operation device.

Therapeutic ultrasound irradiation on the patient 30 from the HIFU transducer unit 12 may be preceded by the following positioning processing. The HIFU control unit 26 causes the ultrasound transducers 2 to transmit ultrasound waves having an intensity lower than that of ultrasound waves used during therapeutic treatment. The HIFU control unit 26 causes the ultrasound probe 10 to scan an ultrasound beam in an observation plane in the patient 30, thereby obtaining B-mode image data. The HIFU control unit 26 causes the display device 24 to display a B-mode image. The user who serves as a treatment provider sees the B-mode image displayed on the display device 24 to check the difference between the location where ultrasound waves transmitted from the HIFU transducer unit 12 are most intense (focal point) and the location of a lesion.

When the difference between the location of the focal point and the location of the lesion falls outside a permissible range, the user operates the robot arm 18 to change the position or attitude of the ultrasound probe 10 and the HIFU transducer unit 12. After confirming that the location of the focal point coincides with the location of the lesion, the user operates the HIFU control unit 26 for therapeutic treatment. In response to user operation, the HIFU control unit 26 causes the ultrasound transducers 2 to transmit therapeutic ultrasound waves having an intensity suitable for therapeutic treatment. A biological tissue at the focal point is thus ablated and subjected to therapeutic treatment.

The HIFU irradiation system 1 according to the illustrated embodiment is used in HIFU therapy performed through treatment sessions in which a lesion is irradiated with ultrasound waves twice with a time interval of, for example, a few days to one week or so. In each of the first and second treatment sessions, multiple spots in the lesion may be irradiated with ultrasound waves. Three-dimensional image data for the lesion is obtained at a point in time before the first treatment session, and at a point in time before the second treatment session (after the first treatment session). First ultrasound image data, which is two-dimensional, is generated from the three-dimensional image data obtained before the first treatment session, and second ultrasound image data, which is two-dimensional, is generated from the three-dimensional image data obtained before the second treatment session. A superimposed image based on the first ultrasound image data and the second ultrasound image data is displayed on the display device 24, and the user compares states of the lesion between before and after the first treatment session and then, for example, sets the irradiation location for the second session.

Three-dimensional image data and a three-dimensional image represented thereby will be described below. FIG. 2 conceptually illustrates a three-dimensional image that is composed of a number N of ultrasound images 40-1 to 40-N. Referring to the example in FIG. 2, each of the ultrasound images 40-1 to 40-N is represented by B-mode image data obtained in an observation plane parallel to the yz plane. Three-dimensional image data that represents a three-dimensional image is a set of pieces of B-mode image data that represent the ultrasound images 40-1 to 40-N.

The HIFU irradiation system 1 generates three-dimensional image data through operation and processing as will be described below. The robot control unit 28 moves the movable assembly 20 along the x axis by a predetermined pitch Δx. Such transport scanning may be performed by moving the movable assembly 20 in the x-axis direction by the pitch Δx while the water bag 14 is being kept in contact with the patient 30. Alternatively, transport scanning may be performed by moving the movable assembly 20 upward so that the water bag 14 moves away from the patient 30, moving the movable assembly 20 in the x-axis direction by the pitch Δx, and then moving the movable assembly 20 downward so that the water bag 14 comes into contact with the patient 30.

The HIFU control unit 26 obtains B-mode image data for N positions on the x axis. Specifically, the HIFU control unit 26 controls the ultrasound probe 10 so that ultrasound beams transmitted from the ultrasound probe 10 are scanned in observation planes parallel to the yz plane, allowing the ultrasound probe 10 to receive ultrasound waves coming from the respective directions of the scanned ultrasound beams. The HIFU control unit 26 generates B-mode image data based on a reception signal output from the ultrasound probe 10 for each of the directions of the scanned ultrasound beams. The HIFU control unit 26 generates three-dimensional image data by obtaining B-mode image data for N positions.

In FIG. 1, the direction away from the head of the patient 30 toward the feet is oriented in the z-axis positive direction, and the upward direction is oriented in the y-axis positive direction. Further, the direction away from the right hand of the patient 30 toward the left hand is oriented in the x-axis positive direction. The relationship between the xyz coordinate system and the posture of the patient 30 illustrated in FIG. 1 is one example, and the relationship between the xyz coordinate system and the posture of the patient 30 may be freely chosen. The foregoing has described processing of generating B-mode image data while the movable assembly 20 is being moved in the x-axis direction by the pitch Δx. Alternatively, the B-mode image data may be generated while the movable assembly 20 is being moved by a predetermined pitch in the y-axis direction or the z-axis direction. Still alternatively, the B-mode image data may be generated while the movable assembly 20 is being moved by a predetermined pitch along a straight line extending in a freely chosen direction that is defined in the xyz coordinate system.

FIG. 3 illustrates a procedure for a first treatment session in the form of a flowchart. A transition from one procedure to the next procedure may be performed in response to user operation or may be automatically performed by the controller 22. Individual procedures may be executed in response to user operation. The HIFU irradiation system 1 generates three-dimensional image data for a lesion that is to be subjected to a first treatment session (S11). The HIFU control unit 26 stores the three-dimensional image data and stores imaging parameters that are set during the generation of the three-dimensional image data (S12). The imaging parameters herein represent a set of variables that are set during generation of individual pieces of B-mode image data that constitute the three-dimensional image data. The imaging parameters include, for example, an amplification degree for a reception signal, a variable that defines image filtering for the B-mode image data, and a variable for adjusting the image quality of the B-mode images. The HIFU irradiation system 1 irradiates the lesion with ultrasound waves for the first treatment session (S13). In the first treatment session, the lesion may be irradiated with therapeutic ultrasound waves multiple times to irradiate multiple spots in the lesion with therapeutic ultrasound waves.

FIG. 4 illustrates a procedure for a second treatment session in the form of a flowchart. A transition from one procedure to the next procedure may be performed in response to user operation or may be automatically performed by the controller 22. Individual procedures may be executed in response to user operation. The HIFU control unit 26 is set to a state for generating three-dimensional image data using the imaging parameters stored before the first treatment session (S21). The robot control unit 28 controls the robot arm 18 to place the movable assembly 20 at a transport scanning start position from where three-dimensional image data has been generated before the first treatment session (S22). The HIFU irradiation system 1 generates three-dimensional image data in the xyz coordinate system in which three-dimensional image data has been generated before the first treatment session (S23).

The HIFU control unit 26 determines the positional relationship between three-dimensional images obtained before and after the first treatment session; in other words, between three-dimensional images obtained before the first treatment session and before the second treatment session (S24). The HIFU control unit 26 determines a correlation value between a first three-dimensional image based on first three-dimensional image data generated before the first treatment session and a second three-dimensional image based on second three-dimensional image data generated before the second treatment session while changing the position of the second three-dimensional image relative to the first three-dimensional image. The HIFU control unit 26 determines a displacement vector for the correlation value that is the maximum as an alignment vector for the three-dimensional images obtained before the first treatment session and before the second treatment session. The displacement vector herein refers to a vector from the second three-dimensional image to the first three-dimensional image.

Based on the alignment vector, the HIFU control unit 26 obtains a first ultrasound image and a second ultrasound image on a common plane passing through the lesion, respectively from the first three-dimensional image and from the second three-dimensional image (S25). Specifically, the HIFU control unit 26 moves the second three-dimensional image in a direction indicated by the alignment vector and by a distance indicated by the alignment vector to obtain B-mode images on the common plane where the lesion appears, as the first ultrasound image and the second ultrasound image.

The HIFU control unit 26 generates a superimposed image having the first ultrasound image and the second ultrasound image laid over each other, and causes the superimposed image to be displayed on the display device 24 (S26). The processing of generating the superimposed image herein may be processing of generating new image data by combining first ultrasound image data and second ultrasound image data with one of the two images being transparent so that the other image can be seen therethrough, to display an image based on the new image data.

The HIFU control unit 26 may cause the superimposed image and one of the first ultrasound image and the second ultrasound image to be displayed adjacent to each other on the display device 24. For the superimposed image that is to be displayed on the display device 24, the HIFU control unit 26 adjusts the transparency of one of the first ultrasound image and the second ultrasound image in response to user operation (S27). The HIFU irradiation system 1 irradiates the lesion with ultrasound waves for the second treatment session (S28).

In the HIFU irradiation system 1 according to an embodiment of the present invention, a HIFU therapeutic display apparatus for use in HIFU therapy is configured as described above. The HIFU therapeutic display apparatus includes the movable assembly 20, the robot arm 18, the controller 22, and the display device 24. The HIFU therapeutic display apparatus executes image obtaining processing of obtaining a first ultrasound image representing a state of the patient 30 before the first treatment session and a second ultrasound image representing a state of the patient 30 before the second treatment session. The HIFU therapeutic display apparatus further executes image generation processing of generating a superimposed image having the first ultrasound image and the second ultrasound image laid over each other and display processing of displaying the superimposed image. The display processing may include processing of displaying the superimposed image and one of the first ultrasound image and the second ultrasound image adjacent to each other. The image generation processing may include processing of adjusting the transparency of one of the first ultrasound image and the second ultrasound image in response to user operation and then generating a superimposed image.

Each of the first ultrasound image and the second ultrasound image is a two-dimensional image generated from a three-dimensional image generated based on ultrasound waves transmitted and received by the ultrasound probe 10 that is transported for scanning. The image obtaining processing is processing of generating, from the three-dimensional image from which the first ultrasound image is generated and the three-dimensional image from which the second ultrasound image is generated, the first ultrasound image and the second ultrasound image on a common plane in the patient 30.

FIG. 5 illustrates, by way of example, an image that is displayed on the display device 24 when the HIFU irradiation system 1 generates three-dimensional image data in step S11 in FIG. 3 and in step S23 in FIG. 4. The display device 24 displays a B-mode image 42 together with a cursor 44. B-mode images 42 that are sequentially generated with lapse of time based on ultrasound waves transmitted and received by the ultrasound probe 10 represent a biological tissue in real time in an observation plane in the patient 30. Referring to the example in FIG. 5, the cursor 44 is composed of a straight line extending in the vertical direction and a straight line extending in the horizontal direction with the point at which the vertical and horizontal straight lines intersect each other being the point pointed by the cursor 44. A prompt 46 "Place the cursor at the center of a lesion. A 3D ultrasound image is produced with this position located at the center." is displayed to the left of the B-mode image 42.

The user operates an operation device connected to the HIFU control unit 26, thereby actuating the robot arm 18 to move the movable assembly 20 so that a lesion 48 appearing in the B-mode image 42 is located at the position of the cursor 44. The HIFU irradiation system 1 generates data for a number N of B-mode images with a predetermined pitch Δ x in the direction of the normal to the image displayed on the display device 24 (x-axis direction) with the position pointed by the cursor 44 being adjusted to be located at the center.

A prompt button 50 indicating "NEXT" and a prompt button 52 indicating "BACK" are given below the prompt 46. Upon clicking on the prompt button 50, the next prompt after the currently displayed prompt is displayed, and upon clicking on the prompt button 52, the next previous prompt before the currently displayed prompt is displayed.

A process indicator 54 is given toward the top of the image. The process indicator 54 shows a procedure for generating three-dimensional image data. Referring to the example in FIG. 5, a procedure including "center setting", "imaging condition setting", "range setting", "move to the start position", "start imaging", and "image confirmation" is given. The process indicator 54 indicates a currently executed step by color or brightness different from that for the other steps. In the "center setting" step, the cursor 44 is placed at the center of the lesion 48. In the "capturing condition setting" step, imaging parameters are set. In the "range setting" step, referring, for example, to the example in FIG. 2, a range in the x-axis direction in which three-dimensional image data is generated is set. In the "move to the start position" step, the movable assembly 20 is moved to the transport scanning start position. In the "start imaging" step, generation of three-dimensional image data is started. In the "image confirmation" step, the user confirms the generated three-dimensional image data through, for example, display of the image.

Such display allows prompts corresponding to respective steps to be displayed sequentially each in response to the user clicking a prompt button every time a step is completed. This enables the user to reliably recognize the operation for generating three-dimensional image data, thereby facilitating the operation for generating three-dimensional image data.

As described above, the HIFU therapeutic display apparatus (the movable assembly 20, the robot arm 18, the controller 22, and the display device 24) sequentially generates B-mode images (ultrasound images) with lapse of time based on ultrasound waves transmitted and received by the ultrasound probe 10. The HIFU therapeutic display apparatus executes adjustment display processing of sequentially displaying the B-mode images sequentially generated with lapse of time together with the cursor 44 (a graphical indication of a reference location of the ultrasound probe 10) with lapse of time.

The HIFU therapeutic display apparatus includes the robot arm 18 serving as the carrier mechanism that is configured to adjust the location (reference location) of the cursor 44 in response to user operation while the adjustment display processing is being executed. Each of the first ultrasound image and the second ultrasound image herein refers to a two-dimensional image that is generated from a corresponding one of three-dimensional images, and the three-dimensional images from which the first ultrasound image and the second ultrasound image are generated are generated based on ultrasound waves transmitted and received by the ultrasound probe 10 that is transported for scanning at the reference location. The HIFU therapeutic display apparatus may display a plurality of process indicators regarding the operation for generating the first ultrasound image or the second ultrasound image, and a prompt for each of the plurality of process indicators.

FIG. 6 illustrates, by way of example, a superimposed image that is displayed at step S26 in FIG. 4. For this superimposed image, a first ultrasound image 60 is laid over a second ultrasound image 62, and the second ultrasound image 62 is seen through the first ultrasound image 60. Each of the first ultrasound image 60 and the second ultrasound image 62 shows a biological tissue 58 that includes a lesion 48. The first ultrasound image 60 shows a lesion 48 which exists before the first treatment session, and the second ultrasound image 62 shows a lesion 48 which exists after the first treatment session, that is, before the second treatment session.

The location of the first ultrasound image 60 relative to the second ultrasound image 62 may be adjusted in accordance with the user's operation. Specifically, the location of the first ultrasound image 60 relative to the second ultrasound image 62 may be adjusted so that the lesions 48 shown in the respective images overlap each other in accordance with the user's operation. The transparency of the first ultrasound image 60 may also be adjusted in accordance with the user's operation.

As shown by the second ultrasound image 62 in FIG. 6, visual identification of the lesion 48 is not easy after the first treatment session, because the lesion 48 is ablated and denatured by therapeutic ultrasound waves. The superimposed image is displayed on the display device 24, thereby facilitating identification of the location of the lesion 48 and the location where the lesion 48 extends. This facilitates the operation of adjusting the location of the focal point of therapeutic ultrasound waves to the location of the lesion 48 for the second treatment session.

In steps S25 and S26 in FIG. 4, the location of a first ultrasound image 60 laid over a second ultrasound image 62 may be adjusted. FIG. 7 illustrates, by way of example, an image that is displayed on the display device 24 while the location of a first ultrasound image 60 is being adjusted. In this example, a first ultrasound image 60 and a second ultrasound image 62 generated in each of three planes located at different positions in the x-axis direction are shown in each of three sections. A prompt 64 "Drag an image to approximately align the images" is displayed to the left of the three sections. The user may drag, for example, the first ultrasound image 60 shown in the lower section of the three sections to adjust the location of the first ultrasound image 60.

A transparency adjustment slider 66 is given below the prompt 64. The transparency adjustment slider 66 is moved from side to side in accordance with the user's cursor operation, thereby adjusting the transparency of the first ultrasound image 60. A reset button 68 is given below the transparency adjustment slider 66. In response to clicking on the reset button 68, the transparency of the first ultrasound image 60 is reset to a predetermined initial value.

A prompt button 70 indicating "NEXT" and a prompt button 72 indicating "BACK" are given below the transparency adjustment slider 66. Upon clicking on the prompt button 70, the next prompt after the currently displayed prompt is displayed, and upon clicking on the prompt button 72, the next previous prompt before the currently displayed prompt is displayed.

A process indicator 74 is given toward the top of the image. Referring to the example in FIG. 7, a procedure including "image selection", "positioning", and "automatic positioning" is given. The process indicator 74 indicates a currently executed step by color or brightness different from that for the other steps. In the "image selection" step, one or more of a plurality of pairs of the first ultrasound image 60 and the second ultrasound image 62 obtained with an interval of Δ x on the x axis are selected in accordance with the user's operation. Referring to the example in FIG. 7, three pairs of the first ultrasound image 60 and the second ultrasound image 62 located at different positions on the x axis are selected, and superimposed images for those pairs are displayed. In the "positioning" step, the location of the first ultrasound image 60 is adjusted in accordance with the user's operation. In the "automatic positioning" step, after the location of the first ultrasound image 60 is adjusted in accordance with the user's operation, the location of the first ultrasound image 60 is automatically adjusted in accordance with the computing processing of the HIFU control unit 26.

As described above, the HIFU therapeutic display apparatus (the movable assembly 20, the robot arm 18, the controller 22, and the display device 24) displays a plurality of process indicators regarding the operation for image generation processing of generating a superimposed image and display processing of displaying the superimposed image, and a prompt for each of the plurality of process indicators.

The automatic positioning is, for example, performed as will be described below. While the first three-dimensional image from which the first ultrasound image 60 is generated is being moved, the HIFU control unit 26 determines a correlation value between the first three-dimensional image from which the first ultrasound image 60 is generated and the second three-dimensional image from which the second ultrasound image 62 is generated. The HIFU control unit 26 generates, from the first three-dimensional image and the second three-dimensional image for the correlation value that is the maximum, a first ultrasound image and a second ultrasound image on a common plane in the patient 30, and causes the display device 24 to display the first ultrasound image and the second ultrasound image.

Such display allows prompts corresponding to respective steps to be displayed sequentially each in response to the user clicking a prompt button every time a step is completed. This enables the user to reliably recognize the operation for displaying a superimposed image, thereby facilitating the operation for displaying a superimposed image.

As illustrated in FIG. 8, the display device 24 may display the first ultrasound image 60 and a superimposed image 76 adjacent to each other for ease of comparison. Referring to the second ultrasound image 62, as described above, visual identification of the lesion 48 is not easy, because the lesion 48 is ablated and denatured by therapeutic ultrasound waves. This may make it difficult for the user to identify a change in state of the lesion 48 resulting from the first treatment session, in turn making it difficult to determine the location that is to be irradiated with therapeutic ultrasound waves in the second treatment session. Having the first ultrasound image 60 and the superimposed image 76 displayed adjacent to each other for ease of comparison makes it easier to determine the location that is to be irradiated with ultrasound waves in the second treatment session. It should be noted that, as an image to be compared with the superimposed image 76, the second ultrasound image 62 may be displayed rather than the first ultrasound image 60.

The display device 24 may display at least one of an image with a graphical indication of an ultrasound irradiation location laid over the first ultrasound image 60 and an image with a graphical indication of an ultrasound irradiation location laid over the second ultrasound image 62. FIG. 9 illustrates an image in which a graphical indication 80 of an ultrasound irradiation location is laid over the first ultrasound image 60. Six spots in the lesion 48 shown in the first ultrasound image 60 are marked by circles serving as the graphical indication 80 of an ultrasound irradiation location. FIG. 10 illustrates an image in which a graphical indication 82 of an ultrasound irradiation location is laid over the second ultrasound image 62. Six spots in the lesion 48 shown in the second ultrasound image 62 are marked by circles serving as the graphical indication 82 of an ultrasound irradiation location.

An image with the graphical indication 80 of an ultrasound irradiation location laid over the first ultrasound image 60 and an image with the graphical indication 82 of an ultrasound irradiation location laid over the second ultrasound image 62 may be displayed on the display device 24 adjacent to each other for ease of comparison. This facilitates determination of the location that is to be irradiated with therapeutic ultrasound waves in the second treatment session.

### REFERENCE SIGNS LIST

1 HIFU irradiation system
2 ultrasound transducer
10 ultrasound probe
12 HIFU transducer unit
14 water bag
16 transducer housing
18 robot arm
18a arm
18b joint
20 movable assembly
22 controller
24 display device
26 HIFU control unit
28 robot control unit
30 patient
40-1 to 40-N ultrasound image
42 B-mode image
44 cursor
46, 64 prompt
48 lesion
50, 52, 70, 72 prompt button
54, 74 process indicator
58 biological tissue
60 first ultrasound image
62 second ultrasound image
66 transparency adjustment slider
68 reset button
76 superimposed image
80, 82 graphical indication of ultrasound irradiation location

## Claims

1. A HIFU therapeutic display apparatus for use in HIFU therapy, the HIFU therapeutic display apparatus being configured to execute:
image obtaining processing of obtaining a first ultrasound image representing a state of a patient before a first treatment session and a second ultrasound image representing a state of the patient before a second treatment session;
image generation processing of generating a superimposed image having the first ultrasound image and the second ultrasound image laid over each other; and
display processing of displaying the superimposed image.

2. The HIFU therapeutic display apparatus according to claim 1, wherein the display processing comprises processing of displaying the superimposed image and one of the first ultrasound image and the second ultrasound image adjacent to each other.

3. The HIFU therapeutic display apparatus according to claim 1 or 2, wherein the image generation processing comprises processing of adjusting transparency of one of the first ultrasound image and the second ultrasound image in response to user operation and then generating the superimposed image.

4. A HIFU therapeutic display apparatus for use in HIFU therapy, the HIFU therapeutic display apparatus being configured to execute:
image obtaining processing of obtaining a first ultrasound image representing a state of a patient before a first treatment session and a second ultrasound image representing a state of the patient before a second treatment session; and
display processing of displaying at least one of an image with a graphical indication of an ultrasound irradiation location laid over the first ultrasound image and an image with a graphical indication of an ultrasound irradiation location laid over the second ultrasound image.

5. The HIFU therapeutic display apparatus according to any one of claims 1 to 4, the HIFU therapeutic display apparatus being configured to:
sequentially generate ultrasound images with lapse of time based on ultrasound waves transmitted and received by an ultrasound probe; and
execute adjustment display processing of sequentially displaying the ultrasound images sequentially generated with lapse of time together with a graphical indication of a reference location of the ultrasound probe with lapse of time,
wherein the HIFU therapeutic display apparatus comprises a carrier mechanism configured to adjust the reference location in response to user operation while the adjustment display processing is being executed,
wherein each of the first ultrasound image and the second ultrasound image is a two-dimensional image generated from a corresponding one of three-dimensional images, and
wherein the three-dimensional images from which the first ultrasound image and the second ultrasound image are generated are generated based on ultrasound waves transmitted and received by the ultrasound probe that is transported for scanning at the reference location.

6. The HIFU therapeutic display apparatus according to claim 5, the HIFU therapeutic display apparatus being configured to display:
a plurality of process indicators regarding operation for generating one of the first ultrasound image and the second ultrasound image; and
a prompt for each of the plurality of process indicators.

7. The HIFU therapeutic display apparatus according to any one of claims 1 to 4,
wherein each of the first ultrasound image and the second ultrasound image is a two-dimensional image generated from a three-dimensional image generated based on ultrasound waves transmitted and received by an ultrasound probe that is transported for scanning, and
wherein the image obtaining processing comprises processing of generating, from the three-dimensional image from which the first ultrasound image is generated and the three-dimensional image from which the second ultrasound image is generated, the first ultrasound image and the second ultrasound image on a common plane in the patient.

8. The HIFU therapeutic display apparatus according to any one of claims 1 to 3, the HIFU therapeutic display apparatus being configured to display:
a plurality of process indicators regarding operation for the image generation processing and the display processing; and
a prompt for each of the plurality of process indicators.
